# EUROPÄISCHE PATENTSCHRIFT

(11) **EP 0 888 296 B1**
(45) Veröffentlichungstag und Bekanntmachung des Hinweises auf die Patenterteilung: **20.12.2000**
(21) Anmeldenummer: 97908265.8
(22) Anmeldetag: 20.03.1997
(51) Int. Cl.: C07C 327/18, C25D 3/46

(54) **IN WASSER SCHWERLÖSLICHE METALLSALZE, VERFAHREN ZUR HERSTELLUNG DERSELBEN UND IHRE VERWENDUNG ALS GLANZZUSATZ ZUR ELEKTROLYTISCHEN ABSCHEIDUNG VON METALLEN**
METALLIC SALTS WHICH ARE DIFFICULT TO DISSOLVE IN WATER, A PROCESS FOR THEIR PRODUCTION, AND THEIR USE AS BRIGHTENERS IN ELECTROLYTIC METAL DEPOSITION
SELS METALLIQUES DIFFICILEMENT SOLUBLES DANS L'EAU, PROCEDE PERMETTANT DE LES PREPARER ET UTILISATION COMME BRILLANTEURS DANS LE DEPOT ELECTROLYTIQUE DE METAUX

(30) Priorität: 23.03.1996 DE 19611565
(43) Veröffentlichungstag der Anmeldung: 07.01.1999
(73) Patentinhaber: BLASBERG OBERFLÄCHENTECHNIK GMBH, 42699 Solingen (DE)
(72) Erfinder: KNAAK, Eberhard, D-40764 Langenfeld (DE); HEYER, Joachim, D-53819 Neunkirchen-Seelscheid (DE); KLEINFELD, Marlies Dr., 42369 Wuppertal (DE); VAN WIJNGAARDEN, Christel, D-42699 Solingen (DE)
(74) Vertreter: Werner, Hans-Karsten, Dr.Dipl.-Chem.
(86) Internationale Anmeldenummer: EP9701389
(87) Internationale Veröffentlichungsnummer: WO9735840

(56) Entgegenhaltungen:
- DE-A- 2 731 595
- DE-C- 885 036
- BULLETIN DE LA SOCIETE CHIMIQUE DE FRANCE, 1962, PARIS, FR, Seiten 2182-2186, XP002033093 A. THUILLIER, ET AL.: "Composés organiques sulfurés. V. - Condensation du sulfure de carbone et de l'acétone"
- BULLETIN DE LA SOCIETE CHIMIQUE DE FRANCE, 1962, PARIS, FR, Seiten 2187-2193, XP002033094 A. THUILLIER, ET AL.: "Composés organiques sulfurés. VI. - Condensation du sulfure de carbone et des cétones aliphatiques"

## Beschreibung

Gegenstand der vorliegenden Erfindung sind in Wasser schwerlösliche Metallsalze der Formel I 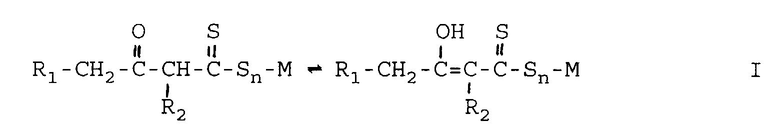 in der R₁ und R₂ gleich oder verschieden sind und Wasserstoff oder eine niedere Alkylgruppe mit 1 bis 3 Kohlenstoffatomen bedeuten, M das Kation eines Metalls ist, welches in Wasser schwerlösliche Sulfide bildet und n 1 oder 2 bedeutet, sowie Gemische derselben, Verfahren zur Herstellung derselben und ihre Verwendung als Glanzzusatz zur elektrolytischen Abscheidung von Metallen.

Aus der DE-PS-885 036 ist ein Verfahren zur Erzeugung glänzender Silberniederschläge bekannt, bei welchem schwefelhaltige Verbindungen wie Schwefelkohlenstoff (und andere schwefelhaltige Verbindungen) mit Ketonen und starkem Alkali umgesetzt und danach angesäuert, ausgefällt und gegebenenfalls nach Wiederauflösung mit Alkali und nach erneuter Ausfällung mit Mineralsäure als Zusatz zu Silberbädern verwendet wurde. Diesen Silberbädern wurden meist noch Formaldehyd oder Türkischrotöl zugesetzt.

Aus der DE-PS 27 31 595 ist bekannt, daß derartige Bäder verbessert werden können, wenn die Kondensationsprodukte aus Keton, Alkalihydroxid und Schwefelkohlenstoff mit wasserlöslichen Salzen von Naphthalinsulfonsäure-Formaldehyd-Kondensationsprodukten versetzt wurden. Insbesondere bei Zusatz zu cyanidischen Silberbädern ist es möglich, so hochglänzende Silberniederschläge herzustellen.

Bereits aus den Arbeiten von Apitzsch Berichte 37, 1599 (1904) und Berichte 38, 2888 (1905) sowie Berichte 41, 4028 (1908) war bekannt, daß bei der Kondensation von Ketonen mit Schwefelkohlenstoff und anschließender Ansäuerung cyclische Verbindungen der Formel 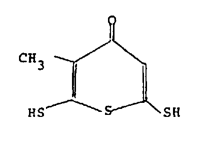 entstehen. Bisher ist man davon ausgegangen, daß diese cyclischen Verbindungen verantwortlich sind für die Glanzbildung in Silberbädern. Massenspektroskopische Untersuchungen von derartigen Reaktionsansätzen haben jetzt bestätigt, daß tatsächlich die cyclischen Verbindungen entstehen. Man hat in der Vergangenheit versucht, insbesondere diese cyclische Fraktion anzureichen und den Glanzbildnern zuzusetzen.

Eingehende Untersuchungen der Anmelderin haben jetzt weiterhin zu dem überraschenden Ergebnis geführt, daß nicht diese cyclischen Verbindungen für die Glanzbildung verantwortlich sind, sondern die als Nebenprodukte vor dem Ansäuern entstehenden Verbindungen der Formel I, in denen jedoch M das Kation eines Metalls ist, welches in Wasser gutlösliche Sulfide bildet. Bei allen bisher beschriebenen und praktizierten Methoden zur Herstellung der Kondensationsprodukte von Ketonen mit Schwefelkohlenstoff sind entweder die wasserlöslichen Alkalisalze oder die durch Säuren ausfällbaren schwerlöslichen cyclischen Verbindungen entstanden.

Es wurde jetzt weiterhin festgestellt, daß diese erfindungsgemäßen Nebenprodukte stabilisiert und in unzersetzter Form abgetrennt werden können, wenn sie als in Wasser schwerlösliche Salze ausgefällt werden. Schwerlösliche Salze bilden vor allem die Metalle, die auch in Wasser schwerlösliche Sulfide bilden. Neben Silber handelt es sich somit insbesondere um Kupfer, Blei, Wismut, Antimon, Cobalt, Eisen, Zink, Palladium, Molybdän, Zinn und Gold.

Es wurde weiterhin festgestellt, daß diese schwerlöslichen Metallsalze nicht nur wesentlich stabiler sind als die bisher als instabil beobachteten Nebenprodukte oder cyclischen Verbindungen, sondern daß darüber hinaus diese schwerlöslichen Metallsalze hervorragend geeignet sind, als Glanzzusatz zu wirken. Dabei kann sogar auf die üblichen Primärglanzbildner wie Türkischrotöl sowie die Kondensationsprodukte von Formaldehyd mit Naphthalinsulfonsäuren verzichtet werden. Auf alle Fälle erhält man mit und ohne Zusatz dieser bekannten primären Glanzbildner erheblich bessere Glanzschichten von Silber. Anscheinend sind diese schwerlöslichen Metallsalze in der Lage, in Elektrolyten soweit wieder in Lösung zu gehen, daß sie die Funktion eines Glanzbildners übernehmen können.

Die neuen Metallsalze sind bereits in Konzentrationen von 0,005 bis 0,3 g/l wirksam. Anscheinend wird durch die übrigen Bestandteile der Elektrolyte die Löslichkeit der schwerlöslichen Metallsalze so erhöht, daß sie wieder in Lösung gehen. Das Verfahren zur Herstellung der schwerlöslichen Metallsalze der Formel I ist dadurch gekennzeichnet, daß Schwefelkohlenstoff mit Ketonen der Formel II 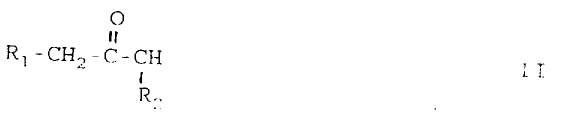 in denen R₁ und R₂ die oben angegebene Bedeutung haben, gegebenenfalls unter Zusatz von nichtwäßrigen, inerten Lösungsmitteln mit starkem Alkali, vorzugsweise Kaliumhydroxid umgesetzt wird, woraufhin durch Zugabe von wäßrigen Lösungen von Metallsalzen der Metalle M, die in Wasser schwerlöslichen Metallsalze der Formel I ausgefällt werden.

Das Mol-Verhältnis zwischen Schwefelkohlenstoff und den Ketonen ist unkritisch. Wichtig ist stets eine ausreichende Menge an Alkalihydroxid. Die Ausfällung der schwerlöslichen Metallsalze ist ebenfalls unproblematisch, da es genügt, die wasserlöslichen Metallsalze in wäßriger Lösung mit dem Reaktionsansatz zu vermischen. Die Mengenverhältnisse und Verfahrensbedingungen scheinen einen gewissen Einfluß zu haben auf das Mengenverhältnis der Substanzen, in denen n = 1 und n = 2 ist. Meist fallen beide Substanzen nebeneinander an. Die in Wasser schwerlöslichen Metallsalze der Formel I fallen aus und können in üblicher Weise abgetrennt werden. Gewünschtenfalls können sie gewaschen und getrocknet werden und in dieser Form auch gelagert und später verwendet werden.

Als Zusatz zu Glanzelektrolyten ist es auch möglich, die roh gefällten Niederschläge ohne weitere Reinigung und Trocknung zu verwenden, da die schwerlöslichen Metallsalze der Formel I auch in dieser Form ausreichend stabil sind.

In den nachfolgenden Beispielen sind die Salze und ihre Herstellung näher erläutert.

### Beispiel 1

1 mol Methylethylketon wird mit 0,65 mol Schwefelkohlenstoff und 1,35 mol Kaliumhydroxid am Rückfluß erhitzt, wodurch die Reaktionstemperatur etwa auf den Siedepunkt von Schwefelkohlenstoff gebracht wird. Nach ca. 2 bis 3 Stunden Reaktionszeit läßt man das Reaktionsgemisch abkühlen, trennt die überstehende flüssige Phase vom Feststoff ab und fällt aus dieser flüssigen Phase mit wäßriger Silbersalzlösung das schwerlösliche Silbersalz aus. Dieses kann gewaschen und getrocknet werden. Der Niederschlag kann auch unmittelbar in 1 %iger cyanidischer Lösung wiederaufgelöst und einem Silberelektrolyten als Glanzbildner zugesetzt werden. Es genügen 0,05 bis 0,3 g/l dieses Silbersalzes als Zusatz zum Glanzelektrolyten.

### Beispiel 2

1 mol Aceton wird mit 0,65 mol Schwefelkohlenstoff und 1,35 mol Kaliumhydroxid umgesetzt. Wie in Beispiel 1 wird aus der flüssigen Phase mit 20 ml Silbernitratlösungen (1 molar) das schwerlösliche Silbersalz abgetrennt. Es wird einem Glanzelektrolyten für Silber zugesetzt, welcher 0,1 bis 1 g/l des Silbersalzes enthält sowie 10 ml/l eines Formaldehyd-Naphthylsulfonsäure-Kondensationsproduktes als primären Glanzbildner.

### Beispiel 3

1 mol Methylethylketon, 0,65 mol Schwefelkohlenstoff und 1,35 mol Kaliumhydroxid werden miteinander umgesetzt. Die flüssige Phase wird mit 25 ml Silbernitratlösung ausgefällt. Der Niederschlag wird in Mengen von 0,01 g/l einem Glanzelektrolyten zugesetzt, welcher 1 ml/l Formaldehyd-Naphthylsulfonsäure-Kondensationsprodukt als primären Glanzbildner enthält.

### Beispiel 4

In analoger Weise wie in Beispiel 3 beschrieben, wird ein Silberglanzelektrolyt hergestellt, welcher mit 10 ml/l Türkischrotöl versetzt ist.

### Beispiel 5

0,15 mol Methylethylketon werden mit 0,6 mol Schwefelkohlenstoff und 0,6 mol KOH umgesetzt. Aus der flüssigen Phase wird mit Silbernitratlösung das Silbersalz ausgefällt.

Zum Vergleich wird der verbleibende Feststoff mit Wasser homogenisiert und mit Säure versehen, wobei freie Säure ausfällt. Der ausgefallene Feststoff wird rasch in Alkalilösung wiederaufgelöst, erneut mit Säure ausgefällt und getrocknet. Der Feststoff wird in Kaliumhydroxid gelöst und als Grundglänzer in cyanidischen Silberelektrolyten zugesetzt zusammen mit dem erfindungsgemäßen Salz.

### Beispiel 6

In einem weiteren Ansatz wird sowohl die flüssige Phase als auch die feste Phase mit Wasser verrührt und dann hieraus mit Silbernitrat das schwerlösliche Silbersalz gefällt.

## Patentansprüche

1. In Wasser schwerlösliche Metallsalze der Formel I 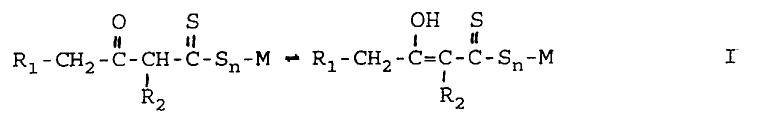 in der R₁ und R₂ gleich oder verschieden sind und Wasserstoff oder eine niedere Alkylgruppe mit 1 bis 3 Kohlenstoffatomen bedeuten, M das Kation eines Metalls ist, welches in Wasser schwerlösliche Sulfide bildet und n 1 oder 2 bedeutet
sowie Gemische derselben.

2. Verfahren zur Herstellung von in Wasser schwerlöslichen Metallsalzen der Formel I 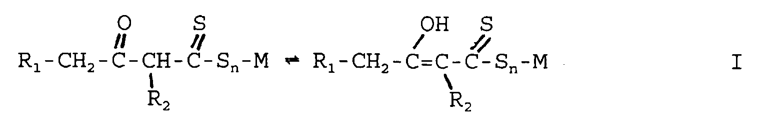 in der R₁ und R₂ gleich oder verschieden sind und Wasserstoff oder eine niedere Alkylgruppe mit 1 bis 3 Kohlenstoffatomen bedeuten, M das Kation eines Metalls ist, welches in Wasser schwerlösliche Sulfide bildet und n 1 oder 2 bedeutet
sowie Gemische derselben,
dadurch gekennzeichnet, daß Schwefelkohlenstoff mit Ketonen der Formel II 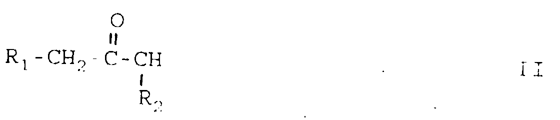 in der R₁ und R₂ die oben genannte Bedeutung haben,
gegebenenfalls unter Zusatz von nichtwäßrigen, inerten Lösungsmitteln und starkem Alkali, vorzugsweise Kaliumhydroxid umgesetzt werden, woraufhin durch Zugabe von wäßrigen Lösungen von Metallsalzen der Metalle M, die in Wasser schwerlöslichen Metallsalze der Formel I ausgefällt werden.

3. Verwendung der in Wasser schwerlöslichen Metallsalze der Formel I als Glanzzusatz zu elektrolytischen Silberbädern.

4. Verwendung gemäß Anspruch 3, dadurch gekennzeichnet, daß das Silbersalz als Glanzzusatz zu elektrolytischen Silberelektrolyten zugesetzt wird.

## Claims

1. Slightly water-soluble metal salts having the formula I 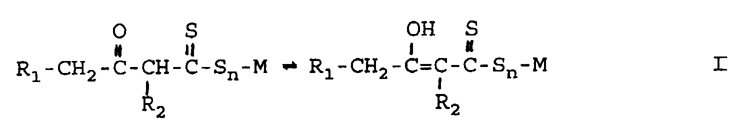 wherein R₁ and R₂ are the same or different and represent hydrogen or a lower alkyl group of from 1 to 3 carbon atoms, M is the cation of a metal which forms slightly water-soluble sulfides, and n means 1 or 2; and mixtures thereof.

2. A process for the preparation of slightly water-soluble metal salts having the formula I 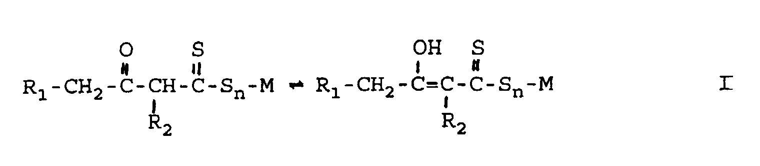 wherein R₁ and R₂ are the same or different and represent hydrogen or a lower alkyl group of from 1 to 3 carbon atoms, M is the cation of a metal which forms slightly water-soluble sulfides, and n means 1 or 2; and mixtures thereof;
characterized in that carbon disulfide is reacted with ketones having the formula II: 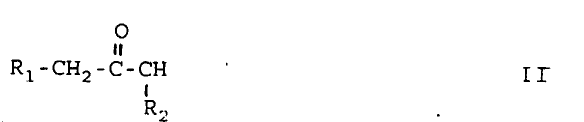 wherein R₁ and R₂ have the same meaning as above, optionally with the addition of non-aqueous inert solvents, in the presence of strong alkali, preferably potassium hydroxide, followed by precipitation of the slightly water-soluble metal salts of formula I by the addition of aqueous solutions of metal salts of the metals M.

3. Use of the slightly water-soluble metal salts of formula I as gloss additives to electrolytic silver plating baths.

4. The use according to claim 3, characterized in that said metal salt which is added to electrolytic silver plating baths as a gloss additive is a silver salt.

## Revendications

1. Sels métalliques peu solubles dans l'eau, de formule I 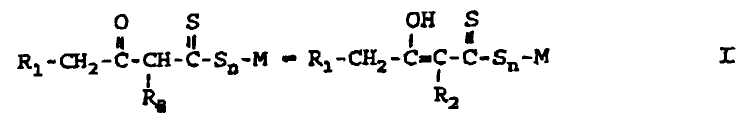 dans laquelle R₁ et R₂ sont identiques ou différents et représentent chacun un atome d'hydrogène ou un groupe alkyle inférieur ayant de 1 à 3 atomes de carbone, M est le cation d'un métal qui forme des sulfures peu solubles dans l'eau, et n vaut 1 ou 2,
ainsi que les mélanges de ces sels.

2. Procédé de préparation de sels métalliques peu solubles dans l'eau, de formule I 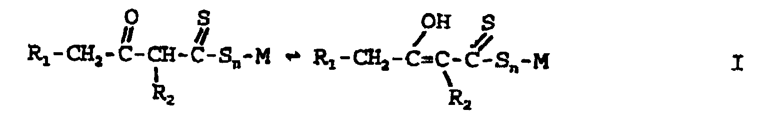 dans laquelle R₁ et R₂ sont identiques ou différents et représentent chacun un atome d'hydrogène ou un groupe alkyle inférieur ayant de 1 à 3 atomes de carbone, M est le cation d'un métal qui forme des sulfures peu solubles dans l'eau, et n vaut 1 ou 2,
ainsi que les mélanges de ces sels,
caractérisé en ce qu'on fait réagir du sulfure de carbone avec des cétones de formule II 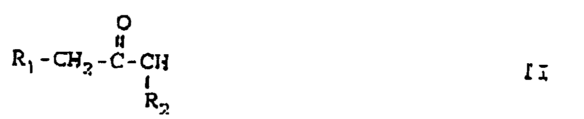 dans laquelle R₁ et R₂ ont les significations données ci-dessus, éventuellement avec addition de solvants inertes non-aqueux et d'une base forte, de préférence l'hydroxyde de potassium, ce après quoi, par addition de solutions aqueuses de sels métalliques des métaux M, on sépare par précipitation les sels métalliques peu solubles dans l'eau de formule I.

3. Utilisation des sels métalliques peu solubles dans l'eau de formule I, en tant qu'additifs de brillantage à des bains à argenter par électrolyse.

4. Utilisation selon la revendication 3, caractérisée en ce que le sel d'argent est ajouté en tant qu'additif de brillantage à des bains à argenter par électrolyse.
